# EUROPEAN PATENT APPLICATION

(11) **EP 4 595 921 A1**
(43) Date of publication of application: **06.08.2025**
(21) Application number: 25155231.1
(22) Date of filing: 31.01.2025
(51) Int. Cl.: A61C 9/00, A61B 1/24, A61C 1/16

(54) **DENTAL SYSTEM**

(71) Applicant: XO Care A/S, 2970 Horsholm (DK)
(72) Inventor: SØRENSEN, Kim, 2970 Hørsholm (DK); SØRENSEN, Peter, 2970 Hørsholm (DK)
(74) Representative: AWA Denmark A/S

(57) **Abstract**

A dental unit for performing dental treatments, the dental unit comprising an instrument bridge for holding one or more dental instruments, wherein the dental instrument bridge is configured to provide electrical power and/or a pressurized fluid to the one or more dental instruments, and wherein the dental unit further comprises an intraoral scanner and wherein the dental unit is configured to provide the intraoral scanner with a pressurized fluid from the dental unit.

## Description

### Field

The present disclosure relates a dental unit for performing dental treatments, an intraoral scanner, and a cap for an intraoral scanner.

### Background

Intraoral scanners have become an important tool for many dental procedures. An intraoral scanner may be used by a dental care professional to create digital impressions of the inside of a patient's mouth. Instead of using traditional impression materials, which can be uncomfortable and messy, the intraoral scanner is a handheld device configured to capture detailed 3D images of the teeth and gums. These digital impressions may then be used to create crowns, bridges, aligners, and other dental appliances with high precision.

Traditionally the intraoral scanner was a wired device connected to a computing device configured to process the recorded sensor signals in order to produce 3D models of the dental cavity.

Lately wireless intraoral scanners have been introduced allowing an improvement in the user-friendliness. Wireless intraoral scanners further make it easier for a dental practice to use the same intraoral scanner together with multiple dental units.

The improvement in user-friendliness together with a reduction in cost has led to a significant increase in use of intraoral scanners.

However, securing a high-quality scan can be a relative time-consuming task for a dental professional as the dental cavity may need to be prepared before initiating the scanning.

Thus, it remains a problem to provide an improved system / intraoral scanner allowing more efficient workflows.

### Summary

According to a first aspect the disclosure relates to a dental unit for performing dental treatments, the dental unit comprising an instrument bridge for holding one or more dental instruments, wherein the dental instrument bridge is configured to provide electrical power, and / or a pressurized fluid to the one or more dental instruments, and wherein the dental unit further comprises an intraoral scanner and wherein the dental unit is configured to provide the intraoral scanner with a pressurized fluid from the dental unit.

Consequently, by providing the intraoral scanner with a pressurized fluid, the intraoral scanner may be used without first having to dry the area to be scanned with a multifunction syringe, thereby improving the quality of the scan and reducing process time.

The pressurize fluid may be compressed air and / or water. The dental unit may comprise a unit configured to pressurize the fluid such as a pump or compressor.

The dental unit may be configured to provide the intraoral scanner with two pressurized fluids e.g., both water and air.

The dental unit may preferable be provided with a dental chair for supporting a patient.

The dental instrument bridge may be configured to provide electrical power, control signals and / or one or more pressurized fluids to one or more of the following dental instruments: a micromotor; a multifunction syringe; a curing light; an air instrument (turbine); a curing light; a scaler; and an intraoral camera.

The system may be provided with one or more valves that can control the flow of the pressurized fluid into the dental cavity such that when the valve is open pressurized fluid is provided to the oral cavity from the intraoral scanner and when the valve is closed no pressurized fluid is provided to the oral cavity. The valves may preferably be provided in the intraoral scanner. Alternatively, the valves may be provided in the dental unit e.g., in the dental instrument bridge. The intraoral scanner may be provided with a control mechanism for controlling the valve such as a button a slider or the like. Alternatively / additionally the dental unit may be provided with a control mechanism for controlling the valve e.g. located at the dental instrument bridge such as a touch display and / or a foot pedal and / or automatically.

The intraoral scanner may be connected to the dental instrument bridge and configured to receive the pressurized fluid from the dental instrument bridge. Alternatively, the intraoral scanner may be connected to another part of the dental unit such as a main support and configured to receive the pressurized fluid therefrom. The dental unit may be provided with a support for supporting the intraoral scanner when not in use. The support may form part of the dental instrument bridge or another part of the dental unit.

In some embodiments, the intraoral scanner comprises a first opening for delivering the pressurized fluid into an oral cavity.

In some embodiments, the intraoral scanner is configured to automatically activate the pressurized fluid and deliver the pressurized fluid into the oral cavity before, during and / or after an intraoral scan.

As an example, the intraoral scanner may be configured to activate the pressurized fluid automatically for a predetermined period of time prior to an intraoral scan to allow the dental care professional to prepare the oral cavity.

In some embodiments, the intraoral scanner is configured to activate the pressurized fluid and deliver the pressurized fluid into the oral cavity during a scan and wherein the intraoral scanner is configured to provide the scan signals to a processing unit configured to process the scan signals recorded while the pressurized fluid is delivered to the oral cavity to detect changes in the shape of parts of the oral cavity indicative of the presence of droplets or other unwanted elements.

Consequently, the user may be provided with feedback of the state of the oral cavity. This may allow the user to cancel an ongoing scan in the event unwanted elements in the oral cavity has been detected, in order to further clean the oral cavity before scanning.

In some embodiments, the pressurized fluid is compressed air and wherein the intraoral scanner is configured to provide the pressurized fluid into the oral cavity for removing liquids from surfaces of the oral cavity.

Consequently, the intraoral scanner may be used to remove droplets from the teeth enabling a higher quality intraoral scan, increase productivity and enhance ergonomics. As an example, in the event a dental care professional discovers during a scan that droplets need to be removed before the scan can be completed, the dental care professional may immediately, using the intraoral scanner as described above to remove the droplets without the need of using another dental instrument.

In some embodiments, the intraoral scanner is configured for cleaning one or more surfaces using the pressurized fluid.

In some embodiments, the intraoral scanner comprises a transparent surface for protecting sensor elements of the intraoral scanner and / or a reflective surface for guiding light and wherein the intraoral scanner is configured to clean the transparent surface and / or the reflective surface using the pressurized fluid.

Consequently, it will be easier to keep the intraoral scanner clean. As an example, if saliva from the patient's mouth reaches a transparent surface of the intraoral scanner, the pressurized fluid may be used to clean the transparent surface in a hygienic and effective way.

In some embodiments, the intraoral scanner has a first part and a second part, the second part being interchangeable connected to the first part, the second part being configured for being at least partly inserted into the oral cavity and wherein intraoral scanner has a channel for delivering the pressurized fluid to the first opening, the channel comprises a first channel part forming part of the first part of the intraoral scanner and a second channel part forming part of the second part of the intraoral scanner, and wherein the first opening is arranged in the second part of the intraoral scanner.

Consequently, by providing a channel in the intraoral scanner divided in a first channel part and a second channel part, for delivering the pressurized fluid to the oral cavity, it may become easier to prepare the intraoral scanner for a new patient by replacing a single part of the intraoral scanner. This may further allow the channel to be integrated as in internal element in the intraoral scanner.

The second channel part may be configured to prevent biological material from a patient to reach the first channel part. This may be achieved by ensuring that the second channel part has a suitable length relative to its diameter. The first part may be configured to allow a user to hold the intraoral scanner.

The first channel part and / or the second channel part may be provided with an engagement element for securing that the fluid may be delivered from the first channel part to the second channel part. The engagement element may be a male / female engagement element, where a distal section of the first channel part is inserted into a proximal section of the second channel part or where a proximal section of the second channel part is inserted into a distal section of the first channel part. Alternatively, the engagement element may be a flanged engagement element, where a distal section of the first channel part is provided with a flange, a proximal section of the second channel element is provide with a flange and where the flange of the first channel part abuts the flange of the second channel part when the first part of the intraoral scanner is connected to the second part of the intraoral scanner.

The first part of the intraoral scanner may be connected to the dental unit via a flexible cable. The first part of the intraoral scanner may be directly connected to the flexible cable or via another part.

The first part of the intraoral scanner may comprise a sensor such as an image sensor for capturing scan data for generating a 3D model of the part of the oral cavity. The second part of the intraoral scanner may comprise a least one transparent surface and / or at least one reflective surface.

In some embodiments, the intraoral scanner comprises a battery and a wireless communication unit allowing the intraoral scanner to be operated wirelessly, and wherein the intraoral scanner can be detached from the dental system when pressurized fluid is not needed.

Consequently, a more flexible system is provided allowing the intraoral scanner to both be operated wireless when there is no need for pressurized fluid and wired when there is a need for pressurized fluid.

In some embodiments, the first part of the intraoral scanner is non permanently connected to a connection part, the connection part being connected to the dental unit via a flexible cable configured to provide the intraoral scanner with the pressurized fluid.

Consequently, the intraoral scanner may be removed from the connection part when it is desired to operate the intraoral scanner wirelessly and connected to the connection part, when it is desired to operate the intraoral scanner wired e.g. when there is a need for pressurised fluid.

In some embodiments, the dental unit is provided with a support configured to support the connection part of the intraoral scanner when the connection part is disconnected from the first part of the intraoral scanner and further the intraoral scanner when the connection part is connected to the first part of the intraoral scanner.

Consequently, it may be ensured that the connection part of the intraoral scanner is supported also when it is unconnected, securing that the working area of the dental care professionals are not affected when the intraoral scanner is operated wireless.

The support may be provided with a first support surface configured to support the connection part when the connection part is disconnected from the first part of the intraoral scanner and a second support surface configured to support the intraoral scanner when connection part is connected to the first part of the intraoral scanner. The support may be part of the instrument bridge or another part of the dental unit.

In some embodiments, the flexible cable is configured to additionally provide the intraoral scanner with electrical power, and wherein the battery of the intraoral scanner can be recharged from the electrical power provided by the flexible cable.

In some embodiments, the flexible cable is further configured to provide the intraoral scanner with control signals for controlling the intraoral scanner. This may allow the dental care professional to control the intraoral scanner using input elements of the dental unit such a foot pedal, a touchscreen, a slider, or a button. The control signals may be used to initiate an intraoral scan and / or change control parameters of the intraoral scanner.

In some embodiments, the flexible cable is configured to receive scanning signals from the intraoral scanner.

In some embodiments, the dental unit comprises a control unit configured to receive scanning signals from the intraoral scanner and transmit scanning signals to a central processing unit configured to process intraoral scanner data.

Consequently, by routing data through the dental unit it may be ensured that the resulting intraoral scans are correctly tied to the patient laying in the dental chair. The signals may be wired or wirelessly transmitted to the dental unit. The signals may be wired transmitted to the dental unit via the flexible cable.

The central processing unit may be provided remote from the dental unit e.g. in a server arranged in the dental practice or remote from the dental practice.

According to a second aspect, the invention relates to an intraoral scanner for a dental unit, wherein the intraoral scanner is configured to receive a pressurized fluid from the dental unit.

In some embodiments, the dental unit is a dental unit as disclosed in relation to the first aspect of the disclosure.

In some embodiments, the intraoral scanner comprises a first opening for delivering the pressurized fluid into an oral cavity.

In some embodiments, the pressurized fluid is compressed air and wherein the intraoral scanner is configured to provide the pressurized fluid into to oral cavity for drying surfaces of the oral cavity.

In some embodiments, the intraoral scanner has a first part and a second part, the second part being interchangeable connected to the first part, the second part being configured for being inserted into the oral cavity, wherein intraoral scanner has a channel for delivering the pressurized fluid to the first opening, the channel comprises a first channel part forming part of the first part of the intraoral scanner and a second channel part forming part of the second part of the intraoral scanner, and wherein the first opening is arranged in the second part of the intraoral scanner.

In some embodiments, the intraoral scanner is configured for cleaning one or more surfaces using the pressurized fluid.

In some embodiments, the intraoral scanner comprises a transparent surface for protecting sensor elements of the intraoral scanner and / or a reflective surface for guiding light and wherein the intraoral scanner is configured to clean the transparent surface and / or the reflective surface using the pressurized fluid.

In some embodiments, the first part of the intraoral scanner is connectable to a connection part, the connection part being connectable to the dental unit via a flexible cable configured to provide the intraoral scanner with the pressurized fluid.

In some embodiments, the intraoral scanner comprises a battery and a wireless communication unit allowing the intraoral scanner to be operated wirelessly, and wherein the intraoral scanner is configured to be detachable from the dental system when pressurized fluid is not needed.

In some embodiments, the intraoral scanner is configured to allow the battery to be re-charged via power provided from a dental unit.

In some embodiments, the intraoral scanner comprises a control unit configured to transmit signals to a central processing unit configured to process intraoral scanner data via a control unit arranged in the dental unit.

According to a third aspect, the invention relates to a cap for an intraoral scanner the cap being connectable to a first part of the intraoral scanner, wherein the cap being configured for being at least partly inserted into the oral cavity when in use and wherein intraoral scanner has a channel for delivering a pressurized fluid to the oral cavity, the channel comprises a first channel part forming part of the first part of the intraoral scanner and a second channel part forming part of the cap, and wherein a first opening is arranged in the cap the first opening being configured to deliver the pressurized fluid into the oral cavity.

The intraoral scanner may be an intraoral scanner as disclosed in relation to the second aspect of the disclosure.

In some embodiments, the cap comprises at least one transparent surface and / or at least one reflective surface and wherein the cap is configured to clean the at least one transparent surface and / or the at least one reflective surface using the pressurized fluid.

The different aspects of the present disclosure can be implemented in different ways including dental units, intraoral scanners, and caps for intraoral scanners described above and in the following, each yielding one or more of the benefits and advantages described in connection with at least one of the aspects described above, and each having one or more preferred embodiments corresponding to the preferred embodiments described in connection with at least one of the aspects described above and/or dis-closed in the dependant claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1 shows a schematic drawing of a dental unit 100 for performing dental treatments according to an embodiment of the present disclosure.
Fig. 2 shows a schematic drawing of an intraoral scanner 102 according to an embodiment of the present disclosure.
Fig. 3 shows a schematic drawing of an intraoral scanner 102 for a dental unit according to an embodiment of the present disclosure.
Fig. 4 shows a cap 200 for an intraoral scanner according to an embodiment of the present disclosure.
Fig. 5 shows a cap 200 for an intraoral scanner according to an embodiment of the present disclosure.
Fig. 6 shows a cap 200 for an intraoral scanner according to an embodiment of the present disclosure.

### Detailed description

In the following description, reference is made to the accompanying figures, which show by way of illustration how the embodiments of the present disclosure may be practiced.

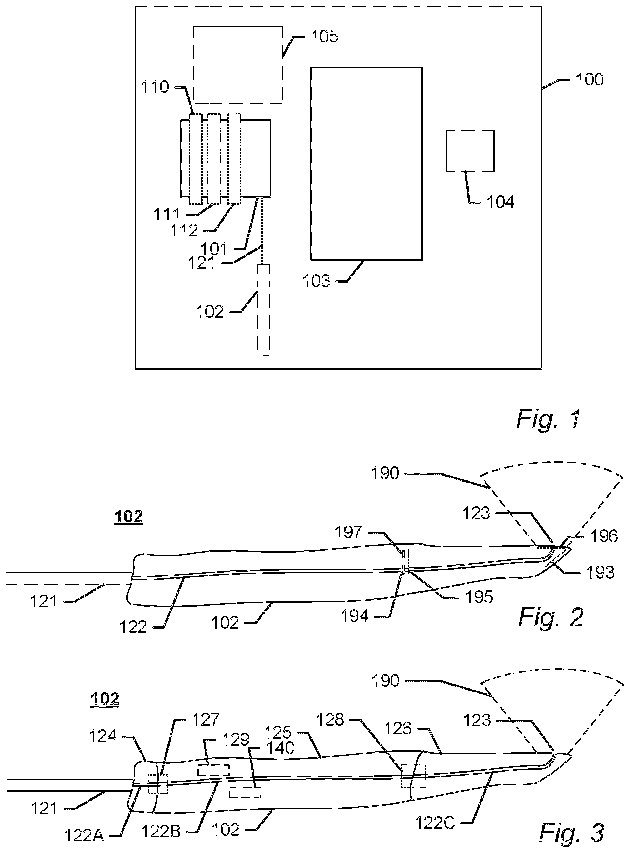

Fig. 1 shows a schematic drawing of a dental unit 100 for performing dental treatments according to an embodiment of the present disclosure. The dental unit 100 comprises an instrument bridge 101 for holding one or more dental instruments 110 111 112. The dental instrument bridge 101 is configured to provide electrical power, control signals and / or a pressurized fluid to the one or more dental instruments 110 111 112.The dental unit further comprises an intraoral scanner 102 and the dental unit is configured to provide the intraoral scanner 102 with a pressurized fluid from the dental unit 100 e.g. the instrument bridge 101.

Consequently, by providing the intraoral scanner with a pressurized fluid, the intraoral scanner may be used directly to dry the oral cavity before scanning for performing a scanning of a high quality without using the multifunction syringe, thereby increasing productivity and enhancing ergonomics. As an example, the pressurized fluid may be a pressurized gas such as atmospheric compressed air that is used to remove droplets in the mouth. Removal of such droplets may enable a better scanning result i.e., resulting in more precise digital impressions. The dental unit 100 may further comprise a dental chair 103 for supporting a patient and one or more displays 104 105 for displaying information relevant for performing a dental procedure.

In this embodiment the intraoral scanner is connected to the dental instrument bridge 101 via a flexible cable 121 and configured to receive the pressurized fluid from the dental instrument bridge 101. However, in other embodiments, the intraoral scanner 102 may be connected to another part of the dental unit 100 such as a main support and configured to receive the pressurized fluid therefrom.

Fig. 2 shows a schematic drawing of an intraoral scanner 102 according to an embodiment of the present disclosure. The intraoral scanner 102 is provided with a sensor 194 such as an image sensor for capturing images within a field of view 190. By moving and rotating the intraoral scanner 102 a plurality of images of a part of the oral cavity may be captured whereby a 3D model of the part of the oral cavity may be formed. The intraoral scanner 102 may further comprise one or more mirrors 193 for guiding light within the intraoral scanner 102. Additionally, the intraoral scanner 102 may comprise one or more transparent surfaces 195 196 for allowing light to travel into the intraoral scanner while preventing biological material from reaching internal compartments of the intraoral scanner 102. The intraoral scanner 102 may further comprise one or more light sources 197 for emitting light into the oral cavity. This may allow the sensor 194 to generate images based on light reflected by to oral cavity originating from the one or more light sources 197. The intraoral scanner 102 is configured to receive a pressurized fluid from a dental unit. The intraoral scanner 102 is configured to be connected to a dental unit via a flexible cable 121. In this embodiment, the intraoral scanner 102 is configured to receive the pressurized fluid from the dental unit via the flexible cable 121. The flexible cable 121 may further provide electrical power and / or control signals to the intraoral scanner 102 and transfer recorded scanning signals to the dental unit. The intraoral scanner 102 has a channel 122 for delivering the pressurized fluid to a first opening 123.

Preferably the channel 122 is an internal channel running internal e.g. a channel arranged within a housing of the intraoral scanner 102. This may make it easier to clean and maintain the intraoral scanner 102.

The intraoral scanner 102 may further be configured to clean one or more of the transparent surfaces 195 196 and / or the reflective surface 193 using a pressurized fluid. This may be done by providing a separate channel (not shown) to one or more of the surfaces 193 195 196 or using the channel 122 possibly by providing a branch to the channel 122 directing the pressurized fluid also to one more of the surfaces 193 195 196. The same pressurized fluid may be used to both clean one or more of the surfaces 193 195 196 and be provided to the opening the first opening 123. Alternatively, a first type of pressurized fluid may be provided to the first opening 123 and another type of pressurized fluid may be used to clean one or more of the surfaces 193 195 196.

Consequently, the intraoral scanner 102 may more easily be cleaned. As an example, if saliva from the patient's mouth reaches the transparent surface 196 of the intraoral scanner 102, a pressurized fluid may be used to clean the transparent surface in a hygienic and effective way e.g. pressurized water and / or air may be used to clean the transparent surface 196.

A cleaning operation of one or more of the surfaces may be done automatically e.g. in a predetermined situation such as before initiating a scan or after having completed a scan. The predetermined situation may occur in connection with each scan, after a predetermined number of scans, or if it is determined that one or more of the surfaces 193 195 196 need to be cleaned. As an example, scan data may be analysed to estimate if one or more of the surfaces 193 195 196 should be cleaned e.g. if a quality parameter of the scan data falls below a threshold.

Additionally / alternatively, a cleaning operation may be performed on demand from the dental professional.

Fig. 3 shows a schematic drawing of an intraoral scanner 102 for a dental unit according to an embodiment of the present disclosure. The intraoral scanner 102 is configured to receive a pressurized fluid from the dental unit. The intraoral scanner 102 has a first part 125 and a second part 126, the second part 126 being interchangeable connected to the first part 125. The second part 126 being configured for being inserted into the oral cavity. The first part 125 being connectable to the dental unit via a flexible cable 121. The intraoral scanner has a channel 122A-C for delivering the pressurized fluid to a first opening 123, the channel 122A-C comprises a first channel part 122B forming part of the first part of the intraoral scanner 125 and a second channel part 122C forming part of the second part of the intraoral scanner 126. The first opening 123 being arranged in the second part of the intraoral scanner 126.

Consequently, by providing a channel in the intraoral scanner 102 divided in a first channel part 122B and a second channel part 122C, for delivering the pressurized fluid to the oral cavity, it may become easier to prepare the intraoral scanner 102 for a new patient by replacing a single part of the intraoral scanner e.g. the second part 126. This may further allow the channel 122A-C to be integrated as in internal element in the intraoral scanner 102.

The first channel part 122B and / or the second channel part 122C may be provided with an engagement element 128 for securing that the fluid may be delivered from the first channel part 122B to the second channel part 122C.

The intraoral scanner 102 may comprise a battery 129 and a wireless communication unit 140 allowing the intraoral scanner 102 to be operated wirelessly. The intraoral scanner 102 may further be configured to be detached from the dental system when pressurized fluid is not needed.

Consequently, a more flexible system is provided allowing the intraoral scanner to both be operated wireless when there is no need for pressurized fluid and wired when there is a need for pressurized fluid.

In this embodiment the first part of the intraoral scanner 125 is non permanently connected to a connection part 124, the connection part 124 being connectable to the dental unit via a flexible cable 121 configured to provide the intraoral scanner 102 with the pressurized fluid. The flexible cable 121 may further provide the intraoral scanner with electrical power, control signals and / or be used to transfer scanning signals from the intraoral scanner to the dental unit. The intraoral scanner may be configured to allow the battery 129 to be recharged via the electrical power provided via the flexible cable 121.

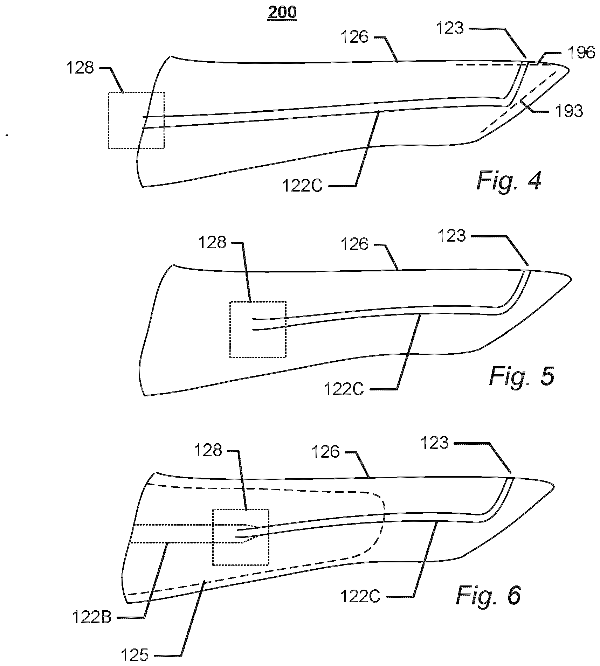

Fig.4 shows a cap 200 for an intraoral scanner according to an embodiment of the present disclosure. The intraoral scanner may be an intraoral scanner as disclosed in relation to Fig. 3 i.e., the cap 200 may correspond to the second element 126 of the intraoral scanner 102. The cap is connectable to a first part of the intraoral scanner and is configured for being at least partly inserted into the oral cavity of a patient when in use. The intraoral scanner has a channel for delivering a pressurized fluid to the oral cavity, the channel comprises a first channel part forming part of the first part of the intraoral scanner and a second channel part 122C forming part of the cap 200, and wherein a first opening 123 is arranged in the cap 200, the first opening 123 being configured to deliver the pressurized fluid into the oral cavity.

The cap 200 may optionally comprise at least one transparent surface 196 and / or at least one reflective surface 193. By providing the cap 200 with at least one transparent surface 196 and / or at least one reflective surface 193 the cap 200 may be designed to protect elements of other parts of the intraoral scanner from substantially coming into contact with biological material during a dental procedure. This may allow the intraoral scanner to be quickly prepared

The second channel part 122C may be provided with an engagement element 128 for securing that the fluid may be delivered from the first channel part to the second channel part. The engagement element 128 may be arranged at an proximal end of the cap 200 as shown in Fig. 4.

However, in another embodiment, as disclosed in Fig. 5 the engagement element 128 may be arranged in a more central position of the cap 200. This may allow a part a first part of the intraoral scanner 125 to be inserted into the cap 200 as illustrated in Fig. 6. In the embodiment of Fig. 6 the engagement element 128 male / female engagement element, where a proximal section of the second channel part 122C is inserted into a distal section of the first channel part 122B.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or de-scribed in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

Further embodiments are disclosed in the following enumerated items.

### Enumerated Items

1. A dental unit for performing dental treatments, the dental unit comprising an instrument bridge for holding one or more dental instruments, wherein the dental instrument bridge is configured to provide electrical power, control signals and / or a pressurized fluid to the one or more dental instruments, and wherein the dental unit further comprises an intraoral scanner and wherein the dental unit is configured to provide the intraoral scanner with a pressurized fluid from the dental unit.
2. A dental unit according to item 1, wherein the intraoral scanner comprises a first opening for delivering the pressurized fluid into an oral cavity.
3. A dental unit according to items 1 or 2, wherein the pressurized fluid is compressed air and wherein the intraoral scanner is configured to provide the pressurized fluid into the oral cavity for removing liquids from surfaces of the oral cavity.
4. A dental unit according to any one of items 1 to 3, the intraoral scanner is configured for drying and cleaning one or more surfaces using the pressurized fluid.
5. A dental unit according to item 4, wherein the intraoral scanner comprises a transparent surface for protecting sensor elements of the intraoral scanner and / or a reflective surface for guiding light and wherein the intraoral scanner is configured to clean the transparent surface and / or the reflective surface using the pressurized fluid.
6. A dental unit according to any one of items 1 to 5, wherein the intraoral scanner has a first part and a second part, the second part being interchangeable connected to the first part, the second part being configured for being at least partly inserted into the oral cavity and wherein intraoral scanner has a channel for delivering the pressurized fluid to the first opening, the channel comprises a first channel part forming part of the first part of the intraoral scanner and a second channel part forming part of the second part of the intraoral scanner, and wherein the first opening is arranged in the second part of the intraoral scanner.
7. A dental unit according to any one of items 1 to 6, wherein the intraoral scanner comprises a battery and a wireless communication unit allowing the intraoral scanner to be operated wirelessly, and wherein the intraoral scanner can be detached from the dental system when pressurized fluid is not needed.
8. A dental unit according to item 7 when dependent on item 6, wherein the first part of the intraoral scanner is non permanently connected to a connection part, the connection part being connected to the dental unit via a flexible cable configured to provide the intraoral scanner with the pressurized fluid.
9. A dental unit according to item 8, wherein the flexible cable is configured to additionally provide the intraoral scanner with electrical power, and wherein the battery of the intraoral scanner can be recharged from the electrical power provided by the flexible cable.
10. A dental unit according to items 8 or 9, wherein the dental unit is provided with a support configured to support the connection part of the intraoral scanner when the connection part is disconnected from the first part of the intraoral scanner and further the intraoral scanner when the connection part is connected to the first part of the intraoral scanner.
11. A dental unit according to item 10, wherein the dental unit comprises a control unit configured to receive signals from the intraoral scanner and transmit signals to a central processing unit configured to process intraoral scanner data.
12. An intraoral scanner for a dental unit according to any one of items 1 to 11, wherein the intraoral scanner is configured to receive a pressurized fluid from the dental unit.
13. An intraoral scanner according to item 12, wherein the intraoral scanner comprises a first opening for delivering the pressurized fluid into an oral cavity.
14. An intraoral scanner according to any one of items 12 to 13, wherein the pressurized fluid is compressed air and wherein the intraoral scanner is configured to provide the pressurized fluid into to oral cavity for drying surfaces of the oral cavity.
15. An intraoral scanner according to any one of items 12 to 14, wherein the intraoral scanner has a first part and a second part, the second part being interchangeable connected to the first part, the second part being configured for being inserted into the oral cavity, wherein intraoral scanner has a channel for delivering the pressurized fluid to the first opening, the channel comprises a first channel part forming part of the first part of the intraoral scanner and a second channel part forming part of the second part of the intraoral scanner, and wherein the first opening is arranged in the second part of the intraoral scanner.
16. An intraoral scanner according to item 15, wherein the first part of the intraoral scanner is connectable to a connection part, the connection part being connectable to the dental unit via a flexible cable configured to provide the intraoral scanner with the pressurized fluid.
17. An intraoral scanner according to any one of items 12 to 16, wherein the intraoral scanner comprises a battery and a wireless communication unit allowing the intraoral scanner to be operated wirelessly, and wherein the intraoral scanner is configured to be detachable from the dental system when pressurized fluid is not needed.
18. An intraoral scanner according to item 17, wherein the intraoral scanner is configured to allow the battery to be re-charged via power provided from a dental unit.
19. An intraoral scanner according to any one of items 12 to 18, wherein the intraoral scanner comprises a control unit configured to transmit signals to a central processing unit configured to process intraoral scanner data via a control unit arranged in the dental unit.
20. An intraoral scanner according to any one of items 12 to 19, wherein the intraoral scanner is configured for cleaning one or more surfaces of the intraoral scanner using the pressurized fluid.
21. An intraoral scanner according to item 20, wherein the intraoral scanner comprises a transparent surface for protecting sensor elements of the intraoral scanner and / or a reflective surface for guiding light and wherein the intraoral scanner is configured to clean the transparent surface and / or the reflective surface using the pressurized fluid.
22. A cap for an intraoral scanner the cap being connectable to a first part of the intraoral scanner, wherein the cap being configured for being at least partly inserted into the oral cavity when in use and wherein intraoral scanner has a channel for delivering a pressurized fluid to the oral cavity, the channel comprises a first channel part forming part of the first part of the intraoral scanner and a second channel part forming part of the cap, and wherein a first opening is arranged in the cap the first opening being configured to deliver the pressurized fluid into the oral cavity.
23. A cap according to item 22, wherein the intraoral scanner is an intraoral scanner according to any one of items 12 to 21.
24. A cap according to any one of items 22 to 23, wherein the cap comprises at least one transparent surface and / or at least one reflective surface and wherein the cap is configured to clean the at least one transparent surface and / or the at least one reflective surface using the pressurized fluid.

## Claims

1. A dental unit for performing dental treatments, the dental unit comprising an instrument bridge for holding one or more dental instruments, wherein the dental instrument bridge is configured to provide electrical power, control signals and / or a pressurized fluid to the one or more dental instruments, and wherein the dental unit further comprises an intraoral scanner and wherein the dental unit is configured to provide the intraoral scanner with a pressurized fluid from the dental unit.

2. A dental unit according to claim 1, wherein the intraoral scanner comprises a first opening for delivering the pressurized fluid into an oral cavity.

3. A dental unit according to claims 1 or 2, wherein the pressurized fluid is compressed air and wherein the intraoral scanner is configured to provide the pressurized fluid into the oral cavity for removing liquids from surfaces of the oral cavity.

4. A dental unit according to any one of claims 1 to 3, the intraoral scanner is configured for drying and cleaning one or more surfaces using the pressurized fluid.

5. A dental unit according to claim 4, wherein the intraoral scanner comprises a transparent surface for protecting sensor elements of the intraoral scanner and / or a reflective surface for guiding light and wherein the intraoral scanner is configured to clean the transparent surface and / or the reflective surface using the pressurized fluid.

6. A dental unit according to any one of claims 1 to 5, wherein the intraoral scanner has a first part and a second part, the second part being interchangeable connected to the first part, the second part being configured for being at least partly inserted into the oral cavity and wherein intraoral scanner has a channel for delivering the pressurized fluid to the first opening, the channel comprises a first channel part forming part of the first part of the intraoral scanner and a second channel part forming part of the second part of the intraoral scanner, and wherein the first opening is arranged in the second part of the intraoral scanner.

7. A dental unit according to any one of claims 1 to 6, wherein the intraoral scanner comprises a battery and a wireless communication unit allowing the intraoral scanner to be operated wirelessly, and wherein the intraoral scanner can be detached from the dental system when pressurized fluid is not needed.

8. A dental unit according to claim 7 when dependent on claim 6, wherein the first part of the intraoral scanner is non permanently connected to a connection part, the connection part being connected to the dental unit via a flexible cable configured to provide the intraoral scanner with the pressurized fluid.

9. A dental unit according to claim 8, wherein the flexible cable is configured to additionally provide the intraoral scanner with electrical power, and wherein the battery of the intraoral scanner can be recharged from the electrical power provided by the flexible cable.

10. A dental unit according to claims 8 or 9, wherein the dental unit is provided with a support configured to support the connection part of the intraoral scanner when the connection part is disconnected from the first part of the intraoral scanner and further the intraoral scanner when the connection part is connected to the first part of the intraoral scanner.

11. A dental unit according to claim 10, wherein the dental unit comprises a control unit configured to receive signals from the intraoral scanner and transmit signals to a central processing unit configured to process intraoral scanner data.

12. An intraoral scanner for a dental unit according to any one of claims 1 to 11, wherein the intraoral scanner is configured to receive a pressurized fluid from the dental unit.

13. An intraoral scanner according to claim 12, wherein the intraoral scanner has a first part and a second part, the second part being interchangeable connected to the first part, the second part being configured for being inserted into the oral cavity, wherein intraoral scanner has a channel for delivering the pressurized fluid to the first opening, the channel comprises a first channel part forming part of the first part of the intraoral scanner and a second channel part forming part of the second part of the intraoral scanner, and wherein the first opening is arranged in the second part of the intraoral scanner.

14. A cap for an intraoral scanner according to claims 12 or 13, wherein the cap being connectable to a first part of the intraoral scanner, wherein the cap being configured for being at least partly inserted into the oral cavity when in use and wherein intraoral scanner has a channel for delivering a pressurized fluid to the oral cavity, the channel comprises a first channel part forming part of the first part of the intraoral scanner and a second channel part forming part of the cap, and wherein a first opening is arranged in the cap the first opening being configured to deliver the pressurized fluid into the oral cavity.

15. A cap according to any one of claims 22 to 23, wherein the cap comprises at least one transparent surface and / or at least one reflective surface and wherein the cap is configured to clean the at least one transparent surface and / or the at least one reflective surface using the pressurized fluid.
